# EUROPEAN PATENT SPECIFICATION

(11) **EP 4 132 495 B1**
(45) Date of publication and mention of the grant of the patent: **25.06.2025**
(21) Application number: 21783888.7
(22) Date of filing: 09.04.2021
(51) Int. Cl.: A61K 31/138, A61P 25/18, A61K 33/14, A61K 31/20, A61K 31/55, A61K 9/28

(54) **ENDOXIFEN FOR THE TREATMENT OF BIPOLAR I DISORDER**
ENDOXIFEN ZUR BEHANDLUNG VON BIPOLARER I-STÖRUNG
ENDOXIFÈNE POUR LE TRAITEMENT D'UN TROUBLE BIPOLAIRE I

(30) Priority: 10.04.2020 US 202063008146 P
(43) Date of publication of application: 15.02.2023
(73) Proprietor: Jina Pharmaceuticals, Inc., Libertyville, IL 60048 (US)
(72) Inventor: AHMAD, Ateeq, Wadsworth, Illinois 60083 (US); AHMAD, Imran, Libertyville, Illinois 60048 (US); AHMAD, Moghisuddin, Wadsworth, Illinois 60083 (US); ALI, Shoukath M, Vernon Hills, Illinois 60061 (US); SHEIKH, Saifuddin, Libertyville, Illinois 60048 (US)
(74) Representative: HGF
(86) International application number: PCT/IB2021/052971
(87) International publication number: WO 2021/205403

(56) References cited:
- WO-A1-2012/050263
- WO-A2-2009/120999
- WO-A2-2014/141292
- US-A1- 2012 164 075
- AHMAD A ET AL: "Endoxifen, a New Treatment Option for Mania: A Double-Blind, Active-Controlled Trial Demonstrates the Antimanic Efficacy of Endoxifen", CTS - CLINICAL AND TRANSLATIONAL SCIENCE, vol. 9, no. 5, 27 June 2016 (2016-06-27), US, pages 252 - 259, XP055855837, ISSN: 1752-8054, Retrieved from the Internet <URL:https://onlinelibrary.wiley.com/doi/full-xml/10.1111/cts.12407> DOI: 10.1111/cts.12407
- SMITH KATHARINE A ET AL: "Lithium and suicide in mood disorders: Updated meta-review of the scientific literature", BIPOLAR DISORDERS, vol. 19, no. 7, 12 September 2017 (2017-09-12), DK, pages 575 - 586, XP093137315, ISSN: 1398-5647, Retrieved from the Internet <URL:https://api.wiley.com/onlinelibrary/tdm/v1/articles/10.1111%2Fbdi.12543> DOI: 10.1111/bdi.12543
- OH JOOYOUNG ET AL: "Risk of developing depression from endocrine treatment: A nationwide cohort study of women administered treatment for breast cancer in South Korea", FRONTIERS IN ONCOLOGY, vol. 12, 20 September 2022 (2022-09-20), CH, XP093137364, ISSN: 2234-943X, DOI: 10.3389/fonc.2022.980197
- KORNREICH CHARLES ET AL: "Suicidal Risk in a Patient Receiving Tamoxifen for Breast Cancer", THE PRIMARY CARE COMPANION, 22 April 2010 (2010-04-22), Retrieved from the Internet <URL:Psychiatrist.com>
- THOMPSON DIANE S. ET AL: "The Relationship Between Tamoxifen, Estrogen, and Depressive Symptoms", BREAST JOURNAL, vol. 5, no. 6, 1 November 1999 (1999-11-01), US, pages 375 - 382, ISSN: 1075-122X, Retrieved from the Internet <URL:https://onlinelibrary.wiley.com/doi/full-xml/10.1046/j.1524-4741.1999.98085.x> DOI: 10.1046/j.1524-4741.1999.98085.x
- CARMASSI CLAUDIA ET AL: "Suicidality and Illness Course Worsening in a Male Patient with Bipolar Disorder during Tamoxifen Treatment for ER+/HER2+ Breast Cancer", CASE REPORTS IN PSYCHIATRY, vol. 2021, 24 March 2021 (2021-03-24), pages 1 - 7, ISSN: 2090-682X, Retrieved from the Internet <URL:http://downloads.hindawi.com/journals/crips/2021/5547649.xml> DOI: 10.1155/2021/5547649
- AHMAD A, SHEIKH S, SHAH T, REDDY MS, PRASAD BSV, VERMA KK, CHANDRAKANT BB, PAITHANKAR M, KALE P, SOLANKI RV, PATEL R, BARKATE H, A: "Endoxifen, a New Treatment Option for Mania: A Double-Blind, Active-Controlled Trial Demonstrates the Antimanic Efficacy of Endoxifen", CTS- CLINICAL AND TRANSLATION SCIENCE, vol. 9, no. 5, 1 October 2016 (2016-10-01), pages 252 - 259, XP055855837, ISSN: 1752-8054, DOI: 10.1111/cts.12407

## Description

### FIELD OF THE INVENTION

The present disclosure relates to a compound for use in a method for decreasing a risk of suicidal behaviour in a bipolar I disorder patient, wherein the patient is undergoing treatment of bipolar I disorder, wherein the compound is endoxifen citrate and the method comprises maintaining the therapeutically effective concentration of endoxifen by administrating a dose of 2 mg to 16 mg of endoxifen citrate in an enteric coated tablet once per day for at least 21 days.

### BACKGROUND OF THE INVENTION

Bipolar disorder is currently a major health problem. Bipolar disorder is a chronic, debilitating illness that affects up to 3 % of the US populations. It causes significant morbidity and imposes a burden on the society. The causes of bipolar disorder are still unknown, and no agent has been specifically developed on the basis of an understanding of the pathophysiology of the illness or mechanism of action for effective treatments.

Several drugs have been approved for treatment of bipolar disorder, such as lithium, valproate or divalproex sodium, carbamazepine, and atypical antipsychotics for the treatment of acute bipolar mania. While these drugs have provided relief for many individuals with bipolar disorder, significant issues with tolerability, efficacy, and attempt suicide or have suicidal behaviour still remain. Further, divalproex has a good tolerability but a high discontinuation rate. There is also a continuous need to observe the therapeutic dose monitoring in patients during the treatment. Short term studies on clinical samples in America showed that early-onset of bipolar I disorder is associated with slow response rate to treatment, persistent mood swings, high recurrence rate, high risk of attempted suicide and severe psychosocial disorders.

The clinicians, for example, may find themselves in situations in which better tolerated agents are less effective, and vice versa. Also, the adherence to the treatment is affected by adverse effects such as sedation, weight gain, alteration in thrombocytopenia, and thyroid disorders.

Study literature reported by Dome *et al.* (2019) bipolar disorders are prevalent mental health illnesses that affect about 1-5 % of the total population, have a chronic course and are associated with a markedly elevated premature mortality. One of the contributors for the decreased life expectancy in bipolar disorders is suicide. The rate of suicide among bipolar disorders patients is approximately 10-30 times higher than the corresponding rate in the general population. Extant research found that up to 20 % of bipolar disorders subjects end their life by suicide, and 20-60 % of them attempt suicide at least one in their lifetime. With a lifetime prevalence of 1.3-5.0 %, type I and type II bipolar disorders are among the most common psychiatric ailments. Patients with bipolar disorders have poor life expectancies as these patients have a decreased lifespan of about 9 - 17 years compared with the general population. Furthermore, some studies from different countries suggest that this mortality gap has become larger over the last decades. Although the largest number of excess death cases in bipolar disorders may be attributed to natural (e.g., due to cardiovascular diseases or diabetes) and not unnatural causes, suicide is also quite prevalent in the population of subjects with bipolar disorders. At a global scale, around 800000 suicide deaths occur every year (which corresponds to a global suicide rate of 11.4/100000/year); thus, suicide may be considered a major public health issue. Although the great majority (≈ 90 %) of suicide cases occur among subjects with major mental-typically mood disorders, the majority of patients with mood disorders never become involved in suicidal behaviour. Accordingly, in addition to major mood disorders, other risk factors (including special clinical features of the mental illness as well as some demographic, personality and familial factors) should contribute to suicidality, which therefore should be deemed as a multi causal phenomenon.

A study reported by Song *et al.* (2017) on around 51000 individuals with bipolar disorder, followed from 2005 to 2013 for treatment with lithium and valproate. Stratified cox regression was used to estimate the hazard ratios of suicide-related events during treated periods compared with untreated periods. For significant associations between medication and suicide-related events, the population attributable fraction was estimated to assess the public health impact for patients with bipolar disorder. During the follow-up, around more than 10000 suicide related events occurred. The incidence rate was significantly decreased by 14 % during lithium treatment but not during valproate treatment. The difference in hazard ratios of suicide related events between lithium and valproate was statistically significant. Estimates of the population attributable fraction suggested that 12 % of suicide related events could have been avoided if patients had taken lithium during the entire study.

Available treatments help a substantial proportion of patients, but are not beneficial for an estimated 40-50 % of patients.

Protein kinase C (PKC) appears to have a role in bipolar disorder. PKC is involved in controlling the function of proteins through the phosphorylation of hydroxyl groups of serine and threonine amino acid residues on these proteins, which are known to play a vital role in cell signalling pathways. It regulates multiple neuronal processes implicated in mood regulation. In current clinical practice, mood stabilizers and antidepressants have been shown to modulate the PKC pathway. Disrupted PKC activity has been found both in post-mortem brains and platelet from patients with mood disorders. Accumulating evidence suggests an imbalance of the PKC signalling system in mood disorders. Thus, PKC is considered as a novel molecular target for the development of innovative medicine for bipolar disorder.

Targeting the PKC signalling pathway for bipolar disorder can improve the patient compliance, when therapeutic dose monitoring is not required in patient, and such treatment can provide significant improvement in mania and depression.

Endoxifen is a non-steroidal selective estrogen receptor modulator (SERM) of the triphenylethylene group. It is an active metabolite of tamoxifen and has been found to be effective in patients that have failed previous hormonal therapies (tamoxifen, aromatase inhibitors, and fulvestrant). The prodrug tamoxifen is metabolized by the CYP2D6 enzyme to produce afimoxifene (4-hydroxytamoxifen) and endoxifen. The chemical name of endoxifen citrate is (Z) - 1 - (4-Hydroxyphenyl) - 1- {4 - [2 -(monomethylamino) ethoxy] phenyl} - 2 - pheny l -1-butene citrate. The empirical formula of endoxifen citrate is C₂₅H₂₇NO₂-C₆H₈O₇, and has following chemical structure as given below (formula I):

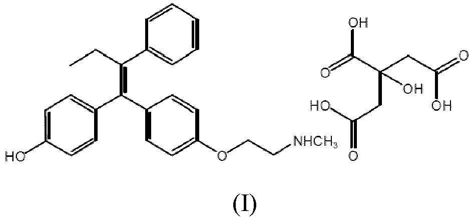

The exact mechanism by which endoxifen exerts its therapeutic effects have not been established in bipolar disorder. However, the efficacy of endoxifen could be mediated through PKC. The PKC represents a family of enzymes highly enriched in brain, where it plays a major role in regulating both pre- and post-synaptic aspects of neurotransmission. Excessive activation of PKC results in symptoms related to bipolar disorder. The PKC signalling pathway is clearly a target for the actions of two structurally dissimilar antimanic agents - lithium and valproate. Tamoxifen, a widely used breast cancer drug is also known to inhibit PKC and demonstrate antimanic properties in human. Endoxifen exhibited four-fold higher potency compared to tamoxifen in inhibiting the PKC activity and is not dependent on the isozyme cytochrome P450 2D6 (CYP2D6) for action on the target tissues. Endoxifen is a PKC inhibitor and is effective in the treatment of bipolar disorder. Further, endoxifen has a broad therapeutic index as compare to divalproex sodium.

A. Ahmad, et al., Clin. Transl. Sci., 2016, 9, 252-259 discloses a double-blind, activecontrolled trial demonstrating the antimanic efficacy of endoxifen.

### SUMMARY OF THE INVENTION

The present invention provides a compound for use in a method for decreasing a risk of suicidal behaviour in a bipolar I disorder patient, wherein the bipolar I disorder patient is undergoing treatment of bipolar I disorder, wherein the compound is endoxifen citrate and wherein the method comprises:
maintaining a therapeutically effective concentration of endoxifen in the patient by administrating a dose of 2 mg to 16 mg of endoxifen citrate in an enteric coated tablet once per day for at least 21 days.

### DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

For the purposes of the present disclosure, any ranges given include both the lower and the upper end points of the range. Ranges given should be considered approximate, unless specifically stated.

The term "C-SSRS" refers to Columbia-Suicide Severity Rating Scale.

The term "PP population" refers to per protocol population to those population who completed the study without major protocol violations/deviations.

The term "EOT" refers to end of treatment.

The term "therapeutically effective concentration" refers to a concentration of endoxifen in plasma which is sufficient to decrease or prevent or cure the symptoms associated with a medical condition or infirmity or to normalize body functions in disease or disorders that result in impairment of specific bodily functions.

The conversion factor for Endoxifen citrate to Endoxifen is 1.514 mg of endoxifen citrate equivalent to 1.000 mg of endoxifen (in free base form).

The term "enteric coating" refers to any pharmaceutically acceptable coating preventing the release of the active agent in the stomach and sufficiently disintegrating in the intestine tract (by contact with approximately neutral or alkaline intestine juices) to allow the resorption of the active agent through the walls of the intestinal tract. The enteric coating remains intact in the acidic environment of the stomach and then solubilize in the more alkaline environment of the small intestine. Generally speaking, enteric coating helps in preventing gastric mucosal irritation and can be used for acid labile drugs which gets denatured in acidic medium.

In an embodiment the patient has manic episodes with or without mixed features.

In another embodiment the patient has depression or has depressive episodes.

In another embodiment the risk of suicidal behavior in the patient is substantially less.

In another embodiment the risk of suicidal behavior in the patient is zero.

In another embodiment the patient had the bipolar I disorder progress during or after treatment with at least one drug(s) selected from lithium, valproate, carbamazepine or an antipsychotic.

In another embodiment the patient had the bipolar I disorder progress during or after treatment with at least one drug(s) selected from lithium, valproate, carbamazepine or an antipsychotic, wherein the risk of suicidal behavior in a patient is substantially less.

In another embodiment the patient had the bipolar I disorder progress during or after treatment with at least one drug(s) selected from lithium, valproate, carbamazepine or an antipsychotic, wherein the risk of suicidal behavior in a patient is zero.

An enteric coated tablet of endoxifen may comprise acryl EZE as a part of the enteric coating composition but the enteric coating is not limited to acryl EZE. The enteric coat can comprise methacrylic acid copolymer (e.g. commercially available Eudragit L and S, Eudragit L 12.5, Eudragit L 100, Eudragit L 100-55, Eudragit L 30 D-55, Eudragit S 12.5 P, Eudragit S 12.5, Eudragit S 100, Eudragit FS 30 D, Acryl-EZE MP), Cellulose acetate phthalate, hydroxypropyl methylcellulose phthalate, Hydroxypropyl methylcellulose acetate succinate, Polyvinyl acetate phthalate, cellulose acetate trimellitate, shellac and the mixture thereof.

### Examples:

The present invention is defined by the claims. It has been described by way of examples only and it is to be recognized that modifications thereto falling within the scope of the appended claims are also considered to be within the scope of this application.

### Example 1:

**Table 1: Formulation details of endoxifen citrate 4mg and 8mg enteric coated tablet.**

| **Sr. No.** | **Ingredients** | **Qty/tab (mg)** | |
|---|---|---|---|
| | | **4mg** | **8mg** |
| **Core Tablet** | | | |
| 1 | Endoxifen Citrate ** | 6.06 | 12.12 |
| 2 | Lactose Monohydrate (PHARMA 200M) DMV | 24.70 | 18.64 |
| 3 | Lactose Monohydrate (DCL 11) | 42.44 | 42.44 |
| 4 | Croscarmellose Sodium | 6.00 | 6.00 |
| 5 | Magnesium Stearate | 0.80 | 0.80 |

| **Seal Coating** | | | |
|---|---|---|---|
| 6 | Hydroxy propyl cellulose (Klucel EF) | 4.00 | 4.00 |
| 7 | Isopropyl Alcohol* | Q.S | Q.S |
| 8 | Purified water* | Q.S | Q.S |

| **Enteric Coating** | | | |
|---|---|---|---|
| 9 | Acryl EZE Yellow 93O82960 | 10.00 | -- |
| 10 | Acryl EZE Green 930510003 | -- | 10.00 |
| 11 | Purified water* | Q.S | Q.S |
| | **Average wt of Coated Tablets (mg)** | 94.00 | 94.00 |

| | | | |
|---|---|---|---|
| Note: Factor for conversion of Endoxifen citrate to Endoxifen: 1.514 mg of Endoxifen citrate is equivalent to 1.000 mg of Endoxifen * - Processing Solvent, shall not present in final formulation except in traces. ** - Actual quantity of Endoxifen Citrate will be based on potency and will be compensated with quantity of Lactose Monohydrate (PHARMA 200M) DMV PH.EUR/USNF. | | | |

### Manufacturing process for example 1:

1. Endoxifen citrate, croscarmellose sodium, lactose monohydrate (Pharma 200M) and lactose monohydrate (DCL) were mixed and sifted together.
2. Magnesium stearate was added to the mixture obtained in step 1, blended and lubricated in a blender.
3. The blend obtained in step 2 was compressed to obtain tablets.
4. A solution of hydroxy propyl cellulose (Klucel EF) was prepared in isopropyl alcohol.
5. An intermediate sub coat was provided on tablets obtained in step 3 using solution obtained in step 4 to obtain sub-coated tablets cores.
6. An enteric coating of acryl EZE was provided on sub-coated tablet core obtained in step 5.

### Clinical study data:

Clinical study of an enteric coated tablet comprising endoxifen citrate formulation was carried out by using example 1 of present application.

### Design of the study:

This study was a multicenter, randomized, double-blind, double-dummy, active controlled, parallel study to assess the efficacy and safety of endoxifen enteric coated tablet 8 mg and divalproex sodium extended release tablet 1000 mg in patient of bipolar I disorders, this study also evaluated the risk of suicidal behavior in the patient and that has progressed during or after treatment with at least one drug(s) selected from lithium, valproate, carbamazepine or an atypical (except clozapine) or typical antipsychotic.

Total 110 patients were qualified for PP population.

The clinical trial study results are incorporated with different parameters as below,

**Table 2: Proportion of patients with C-SSRS score at each visit (PP population)**

| **Day/Visit** | **Questions** | **Results** | **T (N=110)** | **p-value** |
|---|---|---|---|---|
| Day 0 | Q. 1 | YES | 0 (0.00%) | - |
| | | NO | 110 (100.00%) | |
| | Q. 2 | YES | 0 (0.00%) | - |
| | | NO | 110 (100.00%) | |
| Day 7 | Q. 1 | YES | 0 (0.00%) | - |
| | | NO | 110 (100.00%) | |
| | Q. 2 | YES | 0 (0.00%) | - |
| | | NO | 110 (100.00%) | |
| Day 14 | Q. 1 | YES | 0 (0.00%) | - |
| | | NO | 110 (100.00%) | |
| | Q. 2 | YES | 0 (0.00%) | - |
| | | NO | 110 (100.00%) | |
| Day 21 | Q. 1 | YES | 0 (0.00%) | - |
| | | NO | 110 (100.00%) | |
| | Q. 2 | YES | 0 (0.00%) | - |
| | | NO | 110 (100.00%) | |

| | | | | |
|---|---|---|---|---|
| Treatment Specification: T-> Test Product. Note 1: Percentages are calculated based on the total number of patients in each treatment. Note 2: Q. 1 - Have you wished you were dead or wished you could go to sleep and not wake up ? Q. 2 - Have you actually had any thoughts of killing yourself ? p-value could not be calculated as there is no data for response YES in Q. 1 and Q. 2 | | | | |

At day 21 (EOT), endoxifen yielded the risk of suicidal behavior is zero in patient with bipolar I disorders.

Suicidal behavior was not reported during the clinical study.

## Claims

1. A compound for use in a method for decreasing a risk of suicidal behaviour in a bipolar I disorder patient, wherein the bipolar I disorder patient is undergoing treatment of bipolar I disorder, wherein the compound is endoxifen citrate, and wherein the method comprises:
maintaining a therapeutically effective concentration of endoxifen in the patient by administrating a dose of 2 mg to 16 mg of endoxifen citrate in an enteric coated tablet once per day for at least 21 days.

2. The compounds for use of claim 1, wherein the patient has manic episodes with mixed features.

3. The compounds for use of claim 1, wherein the patient has manic episodes without mixed features.

4. The compound for use of claim 1, wherein the patient has depression.

5. The compound for use of claim 1, wherein the patient has depressive episodes.

6. The compound for use of claim 1, wherein the patient had the bipolar I disorder progress during or after treatment with at least one drug(s) selected from lithium, valproate, carbamazepine or an antipsychotic.

## Patentansprüche

1. Verbindung zur Verwendung in einem Verfahren zum Verringern eines Risikos von Selbstmordverhalten bei einem Patienten mit bipolarer I-Störung, wobei der Patient mit bipolarer I-Störung einer Behandlung der bipolaren I-Störung unterzogen wird, wobei die Verbindung Endoxifencitrat ist und wobei das Verfahren Folgendes umfasst:
Aufrechterhalten einer therapeutisch wirksamen Konzentration von Endoxifen bei dem Patienten durch Verabreichen einer Dosis von 2 mg bis 16 mg Endoxifencitrat in einer magensaftresistenten Tablette einmal täglich über mindestens 21 Tage.

2. Verbindungen zur Verwendung nach Anspruch 1, wobei der Patient manische Episoden mit gemischten Merkmalen aufweist.

3. Verbindungen zur Verwendung nach Anspruch 1, wobei der Patient manische Episoden ohne gemischte Merkmale aufweist.

4. Verbindung zur Verwendung nach Anspruch 1, wobei der Patient Depressionen aufweist.

5. Verbindung zur Verwendung nach Anspruch 1, wobei der Patient depressive Episoden aufweist.

6. Verbindung zur Verwendung nach Anspruch 1, wobei der Patient während oder nach einer Behandlung mit mindestens einem Arzneimittel, ausgewählt aus Lithium, Valproat, Carbamazepin oder einem Antipsychotikum das Fortschreiten von bipolarer I-Störung aufwies.

## Revendications

1. Composé destiné à être utilisé dans un procédé de diminution d'un risque de comportement suicidaire chez un patient souffrant d'un trouble bipolaire I, ledit patient souffrant d'un trouble bipolaire I étant en cours de traitement du trouble bipolaire I, ledit composé étant le citrate d'endoxifène, ledit procédé comprenant :
le maintien d'une concentration thérapeutiquement efficace d'endoxifène chez le patient en administrant une dose de 2 mg à 16 mg de citrate d'endoxifène dans un comprimé à enrobage entérique une fois par jour pendant au moins 21 jours.

2. Composés destinés à être utilisés selon la revendication 1, ledit patient souffrant d'épisodes maniaques avec caractéristiques mixtes.

3. Composés destinés à être utilisés selon la revendication 1, ledit patient souffrant d'épisodes maniaques sans caractéristiques mixtes.

4. Composé destiné à être utilisé selon la revendication 1, ledit patient souffrant de dépression.

5. Composé destiné à être utilisé selon la revendication 1, ledit patient souffrant d'épisodes dépressifs.

6. Composé destiné à être utilisé selon la revendication 1, ledit patient ayant présenté une progression du trouble bipolaire I pendant ou après un traitement par au moins un ou plusieurs médicaments choisis parmi le lithium, le valproate, la carbamazépine ou un antipsychotique.
